# EUROPÄISCHE PATENTSCHRIFT

(11) **EP 0 605 933 B1**
(45) Veröffentlichungstag und Bekanntmachung des Hinweises auf die Patenterteilung: **07.10.1998**
(21) Anmeldenummer: 93250360.0
(22) Anmeldetag: 22.12.1993
(51) Int. Cl.: C08J 3/16, C08J 3/05, A61K 9/16, A61K 9/51

(54) **Verfahren zur Herstellung von Pseudolatices und Mikro- oder Nanopartikeln und diese enthaltenden pharmazeutischen Präparaten**
Process for preparing pseudo-latices and micro- or nanoparticles and compositions containing them
Procédé de préparation de pseudo-latex et micro- ou nanoparticules et compositions les contenant

(30) Priorität: 24.12.1992 DE 4244466
(43) Veröffentlichungstag der Anmeldung: 13.07.1994
(73) Patentinhaber: PHARMATECH GmbH, 24220 Flintbek (DE)
(72) Erfinder: Müller, Bernd W., Dr., D-24220 Flintbek (DE); Junis-Specht, Felix, D-24229 Dänisch-Nienhof (DE)
(74) Vertreter: UEXKÜLL & STOLBERG

(56) Entgegenhaltungen:
- DE-A- 3 916 020
- BURI P. ET AL, ED. 'Formes Pharmaceutiques Nouvelles' 1985 , TEC & DOC. LAVOISIER , PARIS, FR Chapter XVI, by Gurny R. * Seite 657 - Seite 671; Tabelle 1 *
- EUROPEAN JOURNAL OF PHARMACEUTICS AND BIOPHARMACEUTICS Bd. 39, Nr. 5 , 1993 , STUTTGART,DE Seiten 173 - 191 XP000403429 ALLEMANN E. ET AL 'Drug-Loaded Nanoparticles - Preparation Methods and Drug Targeting Issues'
- PATENT ABSTRACTS OF JAPAN vol. 012, no. 143 (C-492)30. April 1988 & JP-A-62 262 740 (ICHIMARU FUARUKOSU KK.) 14. November 1987

## Beschreibung

Die Erfindung betrifft ein Verfahren zur Herstellung von Pseudolatices und Mikro- oder Nanopartikeln, deren Verwendung und solche Pseudolatices oder Mikro- oder Nanopartikel umfassende pharmazeutische Präparate.

Mit Pseudolatices werden üblicherweise Systeme bezeichnet, die durch Emulgierung von organischen Polymerlösungen in Wasser und durch Entfernung der Lösungsmittel erhalten werden (G.S. Banker, G.E. Peck, Pharm. Technol. 5 (1981)). Die bisherige Herstellung von Pseudolatices beruht daher hauptsächlich auf einem entsprechend Verfahren, das insbesondere für Cellulosederivate entwickelt wurde (A.M. Ortega, Dissertation Purdue Universität, West Lafayette (USA) (1977)).

Aufgrund der Toxizität, der Kosten und des umweltschädigenden Charakters von organischen Lösungsmitteln wurde versucht, eine Verarbeitung von Polymeren in solchen organischen Lösungsmitteln zu umgehen.

Aus diesem Grunde wurden in der Vergangenheit disperse, wäßrige Systeme einiger Polymere entwickelt, beispielsweise von Cellulosederivaten und Polymethacrylaten.

Dazu wurden verschiedene Wege eingeschlagen:
- Entwicklung von Emulsionspolymerisaten auf Methacrylatbasis (K. Lehmann, D. Dreher, Pharm. Ind. 34, 894 (1972)).
- Teilweiser Ersatz von organischen Lösungsmitteln in wäßrigorganischen Kunststoffemulsionen (K.H. Bauer, H. Osterwald, Pharm. Ind. 41, 1203 (1979)).
- Einsatz von Kunststoffdispersionen zusammen mit wasser- oder alkalilöslichen Hilfsstoffen (W. Rohte, G. Groppenbächer, Pharm. Ind. 34, 892 (1972)).
- Emulgierung in organischen Lösungsmitteln gelöster Cellulosederivate in Wasser und Entfernung der Lösungsmittel (A.M. Ortega, Dissertation Purdue Universität, West Lafayette (USA) (1977)).
- Direkte Emulgierung hydrophiler Methacrylate in Wasser (K. Lehmann, Acta Pharm. Technol. 32, 146 (1986)).
- Dispergierung von mikronisierten Polymeren in wäßrigen Lösungen von Weichmachern und Befilmung bei höheren Temperaturen durch Thermolegierung (K.H. Bauer, H. Osterwald, Acta Pharm. Technol. 27, 99 (1986)).
- Anwendung der wäßrigen Lösungen von Salzen anionischer Polymerisate unter Verwendung flüchtiger Basen (K.H. Frömming, K.P. Krahl, Pharm. Ind. 43, 863 (1981)) oder unter Nachbehandlung mit Säuren (US-A-4 017 647).

Bei niedrigen Temperaturen sind Polymere häufig harte, steife Festkörper. Bei Erwärmung erhält das Polymermaterial soviel thermische Energie, daß seine Ketten bewegen können. Oberhalb der Schmelztemperatur verhält sich das Polymer wie eine viskose Flüssigkeit (vorausgesetzt es erfolgt kein Abbau). Beim Übergang vom festen Glaszustand in den flüssigen Zustand werden verschiedene Zwischenzustände durchlaufen.

Bei kristallinen Polymeren liegt am Schmelzpunkt ein Gleichgewicht zwischen festem und flüssigem Zustand vor. Bei kristallinen Stoffen steigt die Molekularbewegung am Schmelzpunkt von einem relativ niedrigen sprunghaft auf ein hohes Niveau an. Amorphe Polymere verhalten sich dagegen anders. Die Molekularbewegung nimmt hier mit steigender Temperatur in mehreren Stufen langsam zu. Im Gegensatz zu kristallinen Polymeren sind bei amorphen oder teilkristallinen Polymeren nicht zwei, sondern mehrere verschiedene Übergangstemperaturbereiche festzustellen. Oft sind es 5 Viskoelastizitätsbereiche, z.B. bei Polystyrol (J.M.G. Cowie, Chemie und Physik der Polymeren, Verlag Chemie (1976)).

Die Temperatur, bei der ein Polymer vom Glaszustand in den elastischen Zustand übergeht, nennt man Glasübergangstemperatur. Eine viel gebrauchte Methode zur Bestimmung der Glasübergangstemperatur ist die Differentialthermoanalyse (DSC-Methode) (W.C. Stanger, J.K. Guillory, J. Pharm. Sci. 68, 1005 (1979)).

Der Erfindung liegt die Aufgabe zugrunde, ein neues Verfahren zur Herstellung von Pseudolatices und Mikro- oder Nanopartikeln, bei dem insbesondere keine organischen Lösungsmittel verwendet zu werden brauchen und das einfach durchzuführen ist, wobei die Pseudolatices oder Mikro- oder Nanopartikeln wiederum zur Herstellung von pharmazeutischen Präparaten verwendet werden können.

Die erfindungsgemäße Aufgabe wird durch ein Verfahren gelöst, bei dem ein Polymer auf eine Temperatur oberhalb seiner Glasübergangstemperatur erwärmt und anschließend Wasser oder einer wäßrigen Pufferlösung Hochdruck-homogenisiert wird und diese Suspension gegebenenfalls durch Trocknung in Mikro- und/oder Nanopartikeln überführt wird.

Bevorzugte Ausgestaltungen des Verfahrens sind Gegenstand der Unteransprüche.

Pseudolatices können erfindungsgemäß dadurch hergestellt werden, daß bei den Polymeren eine wichtige physikalische Eigenschaft, die "Zustandsänderung", ausgenutzt wird. Solche physikalischen Eigenschaften von Polymeren sind in allgemeiner Form in J. Brandrup und E.H. Immergut (Herausgeber), Polymer Handbuch, 2. Auflage, Wiley, New York (1975); D.W. van Krevelen, Eigenschaften von Polymeren, deren Abschätzung und Korrelierung mit der chemischen Struktur, 2. Auflage, Elsevier, New York (1976) beschrieben.

Der Moment der Zustandsänderung eines Polymers von fester zu flüssiger oder "weicher" Phase kann zur Herstellung von Pseudolatices ausgenutzt werden, indem in dieser Phase eine Homogenisierung erfolgt, wobei es sich vorzugsweise um eine Hochdruckhomogenisierung handelt.

Während bei kristallinen Polymeren nur am Schmelzpunkt oder kurz davor eine Homogenisierung zur Herstellung von Pseudolatices erfolgen kann, können bei amorphen oder teilkristallinen Polymeren verschiedene Temperaturbereiche gewählt werden.

Zu beobachten ist, daß bei amorphen Polymeren wie beispielsweise Polylactiden die Einhaltung niedrigerer Temperaturbereiche zur Herstellung von Pseudolatices mittels Homogenisierung ausreicht, d.h. eine geringe Erweichung des Polymers. Bei Polymeren mit geringeren amorphen Anteilen wie beispielsweise Schellack sind dagegen höhere Temperaturen bzw. Temperaturbereiche erforderlich bzw. einzuhalten.

Als Polymere kommen insbesondere Cellulosederivate, Poly(meth)acrylate, Schellack, Polylactide, Polylactid/Polyglykolid-Mischungen, Polyhydroxybuttersäuren oder Polycyanoacrylate in Betracht.

Die Polymere werden im allgemeinen bezogen auf das wäßrige Medium in einer Menge von 0,01 bis 40 Gew.%, vorzugsweise 0,1 bis 20 Gew.% und bevorzugter 1 bis 15 Gew.% eingesetzt. Am meisten bevorzugt ist eine Menge von 10 Gew.%.

Gegebenenfalls können hohe Glasübergangstemperaturen von Polymeren erniedrigt werden, indem Tenside, Suspensionsstabilisatoren und/oder Weichmachern zugesetzt werden, so daß auch diese Polymere in wäßrigen Systemen zur Herstellung von Pseudolatices verwendet werden können. Sie werden im allgemeinen bezogen auf das wäßrige Medium jeweils in einer Menge von 0,05 bis 10 Gew.%, vorzugsweise 0,1 bis 3 Gew.% und am meisten bevorzugt 0,5 bis 1 Gew.% eingesetzt, wobei die Gesamtmenge 10 Gew.% und vorzugsweise 5 Gew.% nicht überschreitet.

Eine zweite Möglichkeit der Verarbeitung von Polymeren mit hohen Glasübergangstemperaturen besteht beispielsweise in der Erhöhung der Siedetemperatur des wäßrigen Mediums durch Zugabe von Salzen.

Darüber hinaus können weitere übliche Hilfsmittel zugesetzt werden wie beispielsweise Konservierungsmittel, Antioxidantien oder Antischaummittel. Sämtliche Hilfsstoffe können vor der Erwärmung und/oder Homogenisierung oder auch während der Homogenisierung zugesetzt werden.

Das zur Herstellung von Pseudolatices verwendete Polymer kann vor der Erwärmung und Homogenisierung durch pH-Wert-Änderung in eine wasserlösliche Form überführt werden und anschließend aus einer solchen wäßrigen Lösung durch erneute pH-Wert-Änderung wieder ausgefällt werden. So können beispielsweise auch aus sauren Polymeren (z.B. Schellack) Pseudolatices hergestellt werden, indem zuerst eine Fällung des Schellacks mit Säure nach Auflösung in Alkali und dann erst das Erhitzen über den Glaspunkt und die Homogenisierung erfolgt.

Die erfindungsgemäße Herstellung von Pseudolatices und Mikro- und/oder Nanopartikeln erfolgt insbesondere in Abwesenheit organischer Lösungsmittel. Der Einsatz von Tensiden ist hierbei vorteilhaft und erfolgt insbesondere zur Stabilisierung der Suspension. Als Tenside werden vorzugsweise Poloxamere, Lecithine, Schaumverhütungsmittel und Netzmittel eingesetzt.

In das Polymer können ferner Arzneistoffe eingearbeitet werden, so daß wirkstoffhaltige Pseudolatices oder Mikro- oder Nanopartikel hergestellt werden können. Der Arzneistoff wird dazu vor der Homogenisierung in dem Polymer gelöst oder suspendiert. Gemäß einer bevorzugten Ausführungsform wird A) das Polymer auf eine Temperatur oberhalb der Glasübergangstemperatur erwärmt und B) der Arzneistoff (oder mehrere Arzneistoffe) zugesetzt und in dem erweichten Polymer gelöst oder suspendiert. Anschließend kann das arzneistoffhaltige Polymer abkühlengelassen, zerkleinert und gesiebt werden. Die so hergestellten Partikel (Mikro- oder Nanoteilchen) bilden vorzugsweise ein resuspendierbares Pulver. In dieser Form kann es dann in dem erfindungsgemäßen Verfahren zur Herstellung von Pseudolatices und Mikro- oder Nanopartikeln eingesetzt werden. Eine Trocknung, Zerkleinerung und Siebung ist jedoch nicht unbedingt erforderlich. Vielmehr kann das erweichte, arzneistoffhaltige Polymer auch direkt in dem wäßrigen Medium homogenisiert werden.

Falls ein Arzneistoff zugesetzt wird, liegt dieser vorzugsweise in fein gepulverter Form vor. Die Menge an Arzneistoff liegt bezogen auf das Polymer im allgemeinen im Bereich 0,01 bis 40 Gew.%, vorzugsweise 0,1 bis 30 Gew.%, bevorzugter 0,5 bis 20 Gew.% und am meisten bevorzugt im Bereich von 1 bis 15 Gew.%.

Die Partikelgrößen der Pseudolatices liegen im Mikrometer- und/oder Nanometerbereich, wobei sich nach einer Trocknung, beispielsweise Gefrier- oder Sprühtrocknung, Mikro- und/oder Nanopartikel ergeben können.

Die durch Trocknung der homogenisierten Polymersuspension hergestellten Mikro- oder Nanoteilchen sind vorzugsweise ebenfalls ein resuspendierbares Pulver.

Die Oberfläche des Polymers kann ferner durch Hilfsmittel modifiziert werden. Dies erfolgt vorzugsweise durch Sorption von Blockcopolymeren, Proteinen oder Glukoproteinen. Ferner kann das Polymer mit Antikörpern gekoppelt werden. Die Hilfsmittel werden dabei in einer Menge von 1 ng bis 1 mg pro cm² Oberfläche eingesetzt.

Die erfindungsgemäß hergestellten Pseudolatices oder Mikro- oder Nanopartikel eignen sich damit nicht nur ganz allgemein zur Herstellung von pharmazeutischen Präparaten, sondern auch von solchen, bei denen die Wirkstoffe durch Änderung von geeigneten Parametern so gesteuert werden kann, daß eine retardierende Freisetzung des Wirkstoffs möglich ist. Ferner ist eine gezielte Arzneimittelapplikation (drug targeting) möglich.

Die Pseudolatices und Mikro- oder Nanopartikel können selbst arzneimittelfrei sein und als Überzugsmaterialien für Arzneistoffkerne verwendet werden oder sie können eingearbeitete Wirkstoffe umfassen. Insbesondere eignen sich die erfindungsgemäßen Pseudolatices zur Herstellung von Diffusionsfilmen, die beispielsweise für die Herstellung von Filmtabletten verwendet werden können.

Es besteht die Möglichkeit des Einschlusses einer großen Zahl von Wirkstoffen in die Pseudolatices, Mikro- oder Nanopartikel. Bevorzugt können folgende Wirkstoffgruppen mit den Polymeren als Trägermaterialien verarbeitet werden:
- hydroxylierte Kohlenwasserstoffe
- Carbonylverbindungen wir Ketone (z.B. Haloperidol), Monosaccharide, Disaccharide und Aminozucker
- Carbonsäuren wie aliphatische Carbonsäuren, Ester aliphatischer und aromatischer Carbonsäuren, basisch substituierte Ester aliphatischer und aromatischer Carbonsäuren (z.B. Atropin, Scopolamin), Lactone (z.B. Erythromycin), Amide und Imide aliphatischer Carbonsäuren, Aminosäuren, aliphatische Aminocarbonsäuren, Peptide, Polypeptide, β-Lactamderivate, Penicilline, Cephalosporine, aromatische Carbonsäuren (z.B. Acetylsalicylsäure), Amide aromatischer Carbonsäuren, vinyloge Carbonsäuren und vinyloge Carbonsäureester
- Kohlensäurederivate wie Urethane und Thiourethane, Harnstoff und Harnstoffderivate, Guanidinderivate, Hydantoine, Barbitursäurederivate und Thiobarbitursäurederivate
- Nitroverbindungen wie aromatische Nitroverbindungen und heteroaromatische Nitroverbindungen
- Amine wie aliphatische Amin, Aminoglykoside, Phenylalkylamine, Ephedrinderivate, Hydroxyphenylethanolamine, Adrenalinderivate, Amfetaminderivate, aromatische Amine und Derivate und quartäre Aminoverbindungen
- schwefelhaltige Verbindungen wie Thiole und Disulfane, Sulfone, Sulfonsäureester und Sulfonsäureamide
- Polycarbocyclen wie Tetracycline, Steroide mit aromatischem Ring A, Steroide mit α,β-ungesättigter Carbonylfunktion im Ring A und α-Ketol-Gruppe (oder Methylketo-Gruppe) am C₁₇, Steroide mit einem Butenolid-Ring am C₁₇, Steroide mit einem Pentadienolid-Ring am C₁₇ und Seco-Steroide
- O-haltige-Heterocyclen wie Chromanderivate (z.B. Cromoglicinsäure)
- N-haltige Heterocyclen wie Pyrazolderivate (z.B. Propyphenazon, Phenylbutazon), Imidazolderivate (z.B. Histamin, Pilocarpin), Pyridinderivate (z.B. Pyridoxin, Nicotinsäure), Pyrimidinderivate (z.B.Trimethoprim), Indolderivate (z.B. Indometacin), Lysergsäurederivate (z.B. Ergotamin), Yohimbanderivate, Pyrroloindolderivat, Purinderivate (z.B. Allopurinol), Xanthinderivate, 8-Hydroxychinolinderivate, Amino-hydroxy-alkylierte Chinoline, Aminochinoline, Isochinolinderivate (z.B. Morphin, Codein), Chinazolinderivate, Benzopyridazinderivate, Pteridinderivate (z.B. Methotrexat), 1,4-Benzodiazepinderivate, tricyclische N-haltige Heterocyclen, Acridinderivate (z.B. Ethacridin) und Dibenzazepinderivate (z.B. Trimipramin)
- S-haltige Heterocyclen wie Thioxanthenderivate (z.B. Chlorprothixen)
- N,O- und N,S-haltige Heterocyclen wie monocyclische N,O-haltige Heterocyclen, monocyclische N,S-haltige Heterocyclen, Thiadiazinderivate, bicyclische N,S-haltige Heterocyclen, Benzothiadiazinderivate, tricyclische N,S-haltige Heterocyclen und Phenothiazinderivate
- O,N,P-haltige Heterocyclen (z.B. Cyclophosphamid)
- anorganische Verbindungen, wie Ferrofluid (Fe₃O₄), Magnetit und Ionen.

Im allgemeinen werden zur Herstellung von Pseudolatices die Polymere zermörsert, gesiebt (z.B. 90 µm), gegebenenfalls mit Tensiden versetzt und in Wasser dispergiert. Nach der Dispergierung wird, falls gewünscht, Weichmacher zugegeben. Anschließend wird auf auf verschiedene Temperaturen oberhalb der Glasübergangstemperatur erhitzt und hochdruckhomogenisiert.

Die nachfolgenden Beispiele erläutern die Erfindung näher.

### Beispiel 1

### Herstellung von Schellack-Pseudolatices

10 g Blätterschellack werden in 50 ml 50 bis 55°C warmes Wasser gegeben. Unter anteilsweiser Zugabe von festem NaOH (0,6 g) wird die Zubereitung so lange mit einem Magnetrührer (900 UpM) gerührt, bis sich der gesamte Schellack gelöst hat.

Nach Lösen des Schellacks wird unter Rühren (Magnetrührer bei 900 UpM), Ultra-Turrax-Behandlung und anteilsweiser Zugabe von 50 ml 0,15 n-HCl der Schellack gefällt. Die Teilchengrößen betragen bis 100 µm (gemessen mittels Laserbeugungsanalyse, Sympatec, Clausthal-Zellerfeld).

Diese 10 %-ige Schellack-Vordispersion wird mit 1 % Tensid (Pluronic F 68, ICI Surfactants, Cleveland TSJE, England) und wenigen Tropfen Antischaumemulsion (Wacker, Wacker-Chemie GmbH, München) versetzt. Unter starkem Rühren wird bei 500 bar und verschiedenen Temperaturen homogenisiert. Nach der Hochdruckhomogenisation wird die Dispersion kaltgerührt und mittels Laserbeugungsanalyse gemessen.

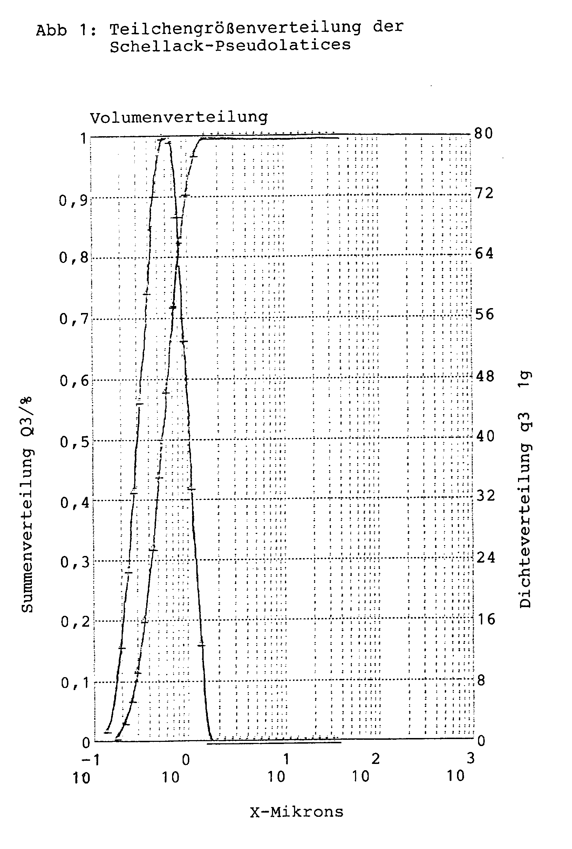

Die Schellack-Pseudolatices zeigten bei den verschiedenen Temperaturen, die bei der Hochdruckhomogenisation jeweils angewendet wurden, verschiedene Teilchengrößenverteilungen. In Tabelle 1 wird deutlich, daß eine Hochdruckhomogenisation ohne jegliche Erwärmung keine Veränderung der Teilchengröße in der jeweiligen Dispersion erbringt. Erst eine Erwärmung bis in den beginnenden Schmelzbereich hinein (Tabelle 1), führte zu einer Verkleinerung der Teilchengrößen. Eine starke Verkleinerung konnte aber erst bei 90°C erreicht werden (s. Volumenverteilung, Figur 1).

Bei Erhöhung des Drucks von 500 auf 1000 bar während der Hochdruckhomogenisation ist eine vernachlässigbare Veränderung der Teilchengröße in den jeweiligen Schellack-Dispersionen festzustellen (Tabelle 2).

Daraus ergibt sich, daß die Temperatur während der Hochdruckhomogenisation einen größeren Einfluß auf die Teilchengröße der Schellack-Pseudolatices besitzt als der Druck.

Die Zetapotentialmessung ergab bei den Schellack-Dispersionen Werte im Bereich von - 42 bis - 47 mV. Daraus resultiert eine beachtliche Abstoßung der Teilchen voneinander, die zur Stabilisierung der hergestellten Dispersionen beiträgt.

### Allgemeines zur Einarbeitung von Wirkstoffen

Das Polymer wird über den Glaspunkt erhitzt und dadurch erweicht. Der Wirkstoff wird in fein gepulverter Form eingearbeitet, wobei er gelöst oder suspendiert in der Polymermatrix vorliegen kann. Nach Einarbeitung des Wirkstoffs wird die Masse gemörsert und gesiebt. Anschließend wird wie oben erwähnt mit der Erwärmung und Homogenisierung fortgefahren.

Die Freisetzung des Wirkstoffs aus dem Polymer hängt einerseits von seiner Wasserlöslichkeit und andererseits von seiner Verteilung im Polymer ab. Ferrofluid (Fe₃O₄) hat beispielsweise die Eigenschaft über mehrere Monate in einer Konzentration von über 90 % in den Pseudolatices (D,L-Polylactid) zu verbleiben.

### Beispiel 2

### Herstellung von wirkstoffhaltigen Polylactidmikropartikeln

Das Polymer (Resomer R 104, Boehringer Ingelheim) wird in einem Kolben im Ölbad bis in den Schmelzbereich hinein erhitzt (80 bis 100°C). Die Diclofenacsäure wird in fein gepulverter Form hinzugefügt und die Zubereitung mittels eines Rührers für 5 Minuten gerührt bis der Arzneistoff in dem Polymer homogen verteilt ist. Nach dem Erkalten und damit der Erhärtung des Polymers wird dieses zerkleinert, gesiebt (90 µm) und mit 1 % (m/m) Tensid (Pluronic F 68) versetzt. Die Zubereitung wird in Wasser dispergiert, auf 60°C erhitzt und bei 500 bar und 1000 bar jeweils zweimal hochdruckhomogenisiert. Die Dispersion wird anschließend kaltgerührt und sofort gefriergetrocknet.

Mittels Laserbeugungsanalyse wird die Teilchengröße bestimmt. Nach Herstellung der arzneistoffbeladenen Mikro- und/oder Nanopartikel erfolgt eine Freisetzungsprüfung des Arzneistoffs in einer Paddle-Apparatur nach USP bei einer Temperatur von 37°C und 100 UpM in Phosphatpuffer (pH 7,4). Die Konzentration des Arzneistoffs wird über 8 Stunden photometrisch ermittelt.

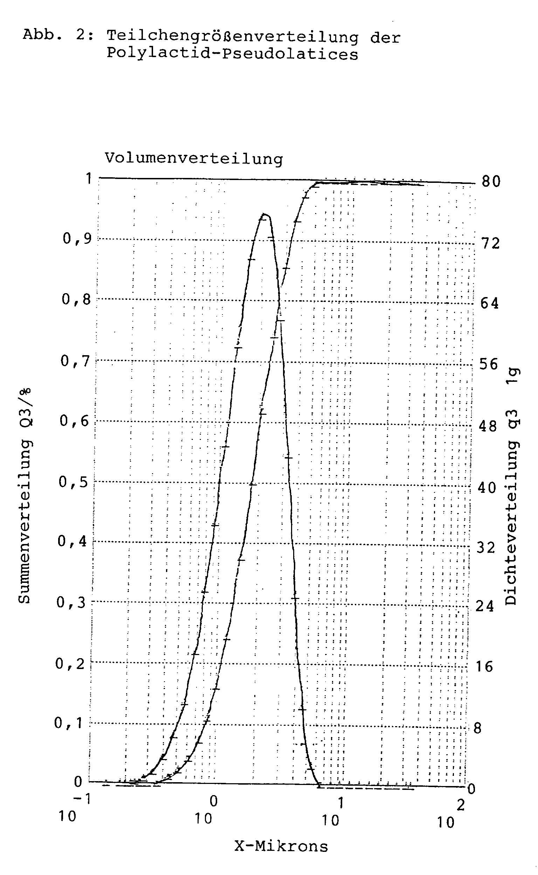

Die Teilchengrößenverteilung beim D,L-Polylactid nach Einarbeitung des Arzneistoffs ist der Figur 2 zu entnehmen. Wie bei Schellack zeigt sich auch hier, daß eine Erhöhung des Drucks keine Verkleinerung der Teilchengröße erbringt.

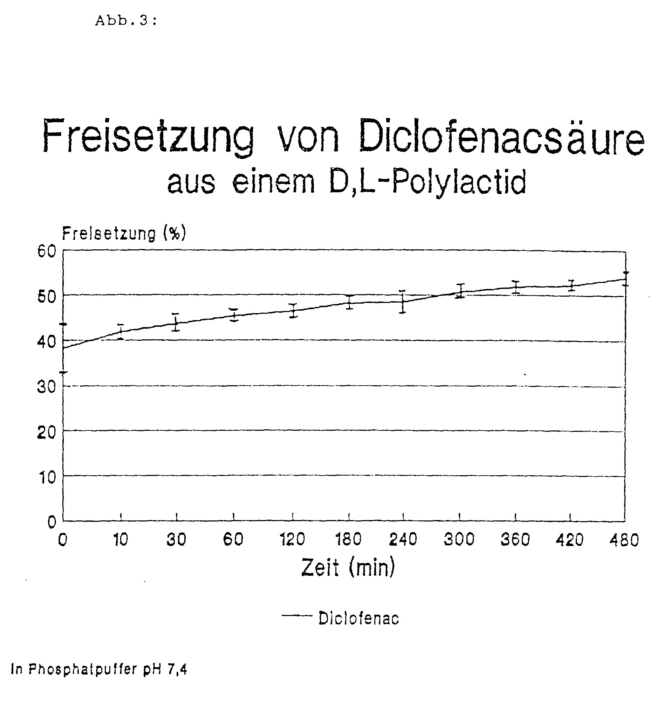

Die Freisetzung des Arzneistoffs aus dem Polylactid zeigt einen stark retardierenden Verlauf (Figur 3). Der unerwünschte Effekt, daß schon zu Anfang eine gewisse Konzentration an Arzneistoff freigesetzt wird, weil sich ein Anteil des Arzneistoffs an der Oberfläche der Partikel befindet, kann durch Waschen der Partikel mit beispielsweise Wasser entfernt werden.

Da bei dieser Methode zur Herstellung von Pseudolatices und Mikro- und/oder Nanopartikeln die Polymere in Wasser dispergiert und über den Glaspunkt bzw. den Schmelzpunkt erhitzt werden, stellt sich als begrenzender Faktor die Siedetemperatur des Wassers dar. Polymere, die eine hohe Glasübergangstemperatur bzw. einen höheren Schmelzpunkt als die Siedetemperatur des Wassers besitzen können auf diese Weise nicht zu Pseudolatices und Mikro- und/oder Nanopartikeln verarbeitet werden. Eine Möglichkeit Polymere mit hohen Glasübergangstemperaturen nach dieser Methode zu verarbeiten ist, wie oben erwähnt, die Zugabe von Weichmachern, die die Eigenschaft besitzen, die Glasübergangstemperatur zu erniedrigen, oder die Zugabe von Salzen, die die Siedetemperatur des Mediums erhöhen.

### Beispiel 3

### Herstellung von Ethylcellulose-Pseudolatices

4,25 g Ethylcellulose, 0,5 g Natriumdodecyclsulfat, 0,5 g Pluronic F 68, 0,5 g Cethylalkohol und 1 g Diethylphthalat werden in 50 ml Wasser gegeben. Nach Erhitzen über 60°C wird anfänglich zweimal bei 100 bar, einmal bei 250 bar und einmal bei 500 bar hochdruckhomogenisiert. Es entstehen Pseudolatices mit einer breiten Teilchengrößenverteilung zwischen 0,1 und 20 µm. Durch die Zugabe von 20 % Diethylphthalat zu der Ethylcellulose wird eine Erniedrigung des Glaspunktes von 125°C auf 60°C erreicht.

**Tabelle 1**

| **Hochdruckhomogenisation bei verschiedenen Temperaturen und Teilchengrößen bei 500 bar** | |
|---|---|
| Temperatur (°C) | Teilchengröße (µm) |
| 20 | bis 100 |
| 60 | bis 40 |
| 70 | bis 32 |
| 80 | bis 28 |
| 90 | bis 2 |

**Tabelle 2**

| **Hochdruckhomogenisation bei verschiedenen Temperaturen und Teilchengrößen bei 100 bar** | |
|---|---|
| Temperatur (°C) | Teilchengröße (µm) |
| 20 | bis 100 |
| 60 | bis 42 |
| 70 | bis 36 |
| 80 | bis 26 |
| 90 | bis 2 |

## Patentansprüche

1. Verfahren zur Herstellung von Pseudolatices und Mikro- und/oder Nanopartikeln, dadurch gekennzeichnet, daß ein Polymer, bei dem es sich um ein Polymer handelt, daß kristallin, teilkristallin oder amorph ist und ausgewählt ist aus Cellulosederivaten, Poly(meth)acrylaten, Schellack, Polylactiden, Polylactid/Polyglycolid-Mischungen, Polyhydroxybuttersäuren oder Polycyanoacrylate, auf eine Temperatur oberhalb seiner Glasübergangstemperatur erwärmt und anschließend in Wasser oder einer wäßrigen Pufferlösung Hochdruck-homogenisiert wird und diese Suspension gegebenenfalls durch Trocknung in Mikro- und/oder Nanopartikel überführt wird.

2. Verfahren nach Anspruch 1, dadurch gekennzeichnet, daß bezogen auf das wäßrige Medium 0,01 bis 30 Gew.% Polymer eingesetzt werden.

3. Verfahren nach Anspruch 1 oder 2, dadurch gekennzeichnet, daß Tenside, Suspensionsstabilisator und/oder Weichmacher zugesetzt werden.

4. Verfahren nach Anspruch 3, dadurch gekennzeichnet, daß die Gesamtmenge an Tensid, Suspensionsstabilisator und Weichmacher bezogen auf das wäßrige Medium im Bereich von 0,05 bis 10 Gew.% liegt.

5. Verfahren nach einem der vorhergehenden Ansprüche, dadurch gekennzeichnet, daß übliche Hilfsstoffe zugesetzt werden.

6. Verfahren nach einem der vorhergehenden Ansprüche, dadurch gekennzeichnet, daß das Polymer vor der Erwärmung und Homogenisierung durch pH-Wert-Änderung in eine wasserlösliche Form überführt anschließend aus einer wäßrigen Lösung durch erneute pH-Wert-Änderung gefällt wird.

7. Verfahren nach einem der vorhergehenden Ansprüche, dadurch gekennzeichnet, daß mindestens ein Arzneistoff zugesetzt wird.

8. Verfahren nach Anspruch 7, dadurch gekennzeichnet, daß der Arzneistoff vor der Homogenisierung in dem Polymer gelöst oder suspendiert wird.

9. Verfahren nach Anspruch 7 oder 8, dadurch gekennzeichnet, daß der Arzneistoff vor der Homogenisierung in das Polymer eingearbeitet wird, indem
a) das Polymer auf eine Temperatur oberhalb der Glasübergangstemperatur erwärmt wird,
b) der Arzneistoff zugesetzt und in dem erweichten Polymer gelöst oder suspendiert wird und
c) das Arzneistoff enthaltende Polymer abkühlen gelassen, zerkleinert und gesiebt wird.

10. Verfahren nach einem der Ansprüche 7 bis 9, dadurch gekennzeichnet, daß der Arzneistoff in fein gepulverter Form eingesetzt wird.

11. Verfahren nach einem der vorhergehenden Ansprüche, dadurch gekennzeichnet, daß bei der Herstellung von Mikro- und/oder Nanopartikeln die Trocknung durch Gefrier- oder Sprühtrocknung erfolgt.

12. Verfahren nach einem der vorhergehenden Ansprüche, dadurch gekennzeichnet, daß die Oberfläche des Polymers durch Hilfsstoffe modifiziert wird.

13. Verfahren nach Anspruch 12, dadurch gekennzeichnet, daß das die Oberflächenmodifizierung durch Sorption von Blockcopolymeren, Proteinen oder Glucoproteinen erfolgt.

14. Verfahren nach einem der vorhergehenden Ansprüche, dadurch gekennzeichnet, daß das Polymer mit Antikörpern gekoppelt wird.

15. Teilchen, deren Teilchengröße im Mikro- oder Nanometerbereich liegt und die ein Polymer umfassen, das kristallin, teilkristallin oder amorph ist und ausgewählt ist aus Cellulosederivaten, Poly(meth)acrylaten, Schellack, Polylactiden, Polylactid/Polyglycolid-Mischungen, Polyhydroxybuttersäuren oder Polycyanoacrylaten, und herstellbar sind indem das Polymer auf eine Temperatur oberhalb seiner Glasübergangstemperatur erwärmt und anschließend in Wasser oder einer wäßrigen Pufferlösung Hochdruck-homogenisiert wird und diese Suspension durch Trocknung in Mikro- und/oder Nanopartikel überführt wird.

16. Pseudolatices, die Teilchen enthalten, deren Teilchengröße im Mikro- oder Nanometerbereich liegt und die ein Polymer umfassen, das kristallin, teilkristallin oder amorph ist und ausgewählt ist aus Cellulosederivaten, Poly(meth)acrylaten, Schellack, Polylactiden, Polylactid/Polyglycolid-Mischungen, Polyhydroxybuttersäuren oder Polycyanoacrylaten, und herstellbar sind indem das Polymer auf eine Temperatur oberhalb seiner Glasübergangstemperatur erwärmt und anschließend in Wasser oder einer wäßrigen Pufferlösung Hochdruck-homogenisiert wird.

17. Verwendung von Pseudolatices oder Mikro- und/oder Nanopartikeln hergestellt gemäß einem der Ansprüche 1 bis 15 zur Herstellung von pharmazeutischen Präparaten.

18. Verwendung nach Anspruch 17, dadurch gekennzeichnet, daß in die Pseudolatices, Mikro- und/oder Nanopartikel Arzneistoffe eingearbeitet werden.

19. Verwendung nach Anspruch 17 oder 18, dadurch gekennzeichnet, daß die pharmazeutischen Präparate Diffusionsfilme sind.

## Claims

1. Process for the production of pseudolatices and micro- and/or nanoparticles, characterized in that a polymer, being a polymer which is crystalline, partially crystalline or amorphous and is selected from cellulose derivatives, poly(meth)acrylates, shellac, polylactides, polylactide/polyglycolide mixtures, polyhydroxybutyric acids or polycyanoacrylates, is heated to a temperature above its glass transition temperature and then highpressure homogenized in water or an aqueous buffer solution and this suspension is optionally converted by drying into micro- and/or nanoparticles.

2. Process according to claim 1, characterized in that 0.01 to 30 wt.% polymer are used relative to the aqueous medium.

3. Process according to claim 1 or 2, characterized in that surfactants, suspension stabilizers and/or plasticizers are added.

4. Process according to claim 3, characterized in that the total quantity of surfactant, suspension stabilizer and plasticizer lies in the range from 0.05 to 10 wt.%, relative to the aqueous medium.

5. Process according to one of the preceding claims, characterized in that usual auxiliaries are added.

6. Process according to one of the preceding claims, characterized in that , prior to heating and homogenization, the polymer is converted into a watersoluble form by changing the pH value and then precipitated from an aqueous solution by a fresh change of pH value.

7. Process according to one of the preceding claims, characterized in that at least one medicament is added.

8. Process according to claim 7, characterized in that the medicament is dissolved or suspended in the polymer prior to homogenization.

9. Process according to claim 7 or 8, characterized in that the medicament is incorporated into the polymer prior to homogenization by
a) heating the polymer to a temperature above the glass transition temperature,
b) adding the medicament and dissolving or suspending it in the softened polymer and
c) allowing the medicament-containing polymer to cool, comminuting it and sieving it.

10. Process according to one of claims 7 to 9, characterized in that the medicament is used in finely powdered form.

11. Process according to one of the preceding claims, characterized in that, in the production of micro- and/or nanoparticles, drying is carried out by freeze- or spray-drying.

12. Process according to one of the preceding claims, characterized in that the surface of the polymer is modified by auxiliaries.

13. Process according to claim 12, characterized in that the superficial modification takes place through sorption of block copolymers, proteins or glucoproteins.

14. Process according to one of the preceding claims, characterized in that the polymer is coupled with antibodies.

15. Particles whose particle size lies in the micro- or nanometer range and which comprise a polymer which is crystalline, partially crystalline or amorphous and is selected from cellulose derivatives, poly(meth)acrylates, shellac, polylactides, polylactide/ polyglycolide mixtures, polyhydroxy- butyric acids or polycyanoacrylates, and can be prepared by heating the polymer to a temperature above its glass transition temperature and subsequently subjecting it to highpressure homogenization in water or an aqueous buffer solution and converting this suspension into micro- and/or nanoparticles by drying.

16. Pseudolatices which contain particles whose particle size lies in the micro- or nanometer range and which comprise a polymer which is crystalline, partially crystalline or amorphous and is selected from cellulose derivatives, poly(meth)acrylates, shellac, polylactides, polylactide/ poly-glycolide mixtures, polyhydroxybutyric acids or polycyanoacrylates, and can be prepared by heating the polymer to a temperature above its glass transition temperature and subsequently subjecting it to highpressure homogenization in water or an aqueous buffer solution.

17. Use of pseudolatices or micro- and/or nanoparticles produced according to one of claims 1 to 15 for the production of pharmaceutical preparations.

18. Use according to claim 17, characterized in that medicaments are worked into the pseudolatices, micro- and/or nanoparticles.

19. Use according to claim 17 or 18, characterized in that the pharmaceutical preparations are diffusion films.

## Revendications

1. Procédé pour la production de pseudoréseaux et de micro et/ou nanoparticules, caractérisé en ce qu'un polymère pour lequel il s'agit d'un polymère qui est cristallin, partiellement cristallin ou amorphe et qui est choisi parmi des dérivés de cellulose, des poly(meth)acrylates, du shellac, des polylactides, des mélanges de polylactides/polyglycolides, des acides polyhydroxybutyriques ou des polycynaoacrylates est chauffé à une température au-dessus de sa température de transition vitreuse et est ensuite homogénéisé à haute pression dans l'eau ou une solution tampon aqueuse et cette suspension est le cas échéant transformée par séchage en micro et/ou nanoparticule.

2. Procédé selon la revendication 1, caractérisé en ce que, en se rapportant au milieu aqueux, on utilise 0,01 à 30% en poids du polymère.

3. Procédé selon la revendication 1 ou 2, caractérisé en ce que des agents tensio-actifs, un stabilisant de la suspension et/ou un adoucissant sont ajoutés.

4. Procédé selon la revendication 3, caractérisé en ce que la quantité totale de l'agent tensio-actif du stabilisant, de la suspension et de l'adoucissant, rapportée au milieu aqueux, est dans la gamme de 0,05 à 10% en poids.

5. Procédé selon l'une des revendications précédentes, caractérisé en ce que des auxiliaires habituels sont ajoutés.

6. Procédé selon l'une des revendications précédentes, caractérisé en ce que le polymère est transformé avant chauffage et homogénéisation, par modification de la valeur du, pH en une forme soluble dans l'eau et ensuite il est précipité d'une solution aqueuse par nouvelle modification de la valeur du pH.

7. Procédé selon l'une des revendications précédentes, caractérisé en ce qu'au moins un médicament est ajouté.

8. Procédé selon la revendication 7, caractérisé en ce que le médicament est dissous ou mis en suspension dans le polymère avant l'homogénéisation.

9. Procédé selon la revendication 7 ou 8, caractérisé en ce que le médicament est introduit dans le polymère avant l'homogénéisation par le fait que
a) le polymère est chauffé à une température au-dessus de la température de transition vitreuse,
b) le médicament est ajouté et est dissous dans le polymère ramolli ou bien y est mis en suspension et
c) le polymère contenant le médicament que l'on a laissé refroidir, est broyé et tamisé.

10. Procédé selon l'une des revendications 7 à 9, caractérisé en ce que le médicament est utilisé en forme de poudre fine.

11. Procédé selon l'une des revendications précédentes, caractérisé en ce que dans la production de micro et/ou nanoparticules, le séchage se produit par lyophilisation ou séchage par pulvérisation.

12. Procédé selon l'une des revendications précédentes, caractérisé en ce que la surface du polymère est modifiée par des auxiliaires.

13. Procédé selon la revendication 12, caractérisé en ce que la modification de surface se produit par sorption de copolymères séquencés, protéines ou glycoprotéines.

14. Procédé selon l'une des revendications précédentes, caractérisé en ce que le polymère est couplé à des anticorps.

15. Particules, dont la dimension de particule se trouve dans le domaine des micro ou nanomètres et qui contiennent un polymère qui est cristallin, partiellement cristallin ou amorphe et est choisi parmi des dérivés de cellulose, des poly(meth)acrylates, du shellac, des polylactides, des mélanges de polylactides/polyglycolides, des acides polyhydroxybutyriques ou des polycyanoacrylates, et qui peuvent être fabriquées par le fait que le polymère est chauffé à une température au-dessus de sa température de transition vitreuse et est ensuite homogénéisé à haute pression dans l'eau ou dans une solution tampon aqueuse et cette suspension est transformée par séchage en micro et/ou nanoparticule.

16. Pseudo-réseaux qui contiennent des particules dont la dimension de particule se trouve dans la gamme des micro- ou nanomètres et qui contiennent un polymère qui est cristallin, partiellement cristallin ou amorphe et est choisi parmi des dérivés de cellulose, des poly(meth)acrylates, du shellac, des polylactides, des mélanges de polylactides/polyglycolides, des acides polyhydroxybutyriques ou des polycyanoacrylates, et qui peuvent être fabriquées par le fait que le polymère est chauffé à une température au-dessus de sa température de transition vitreuse et est ensuite homogénéisé à haute pression dans l'eau ou dans une solution tampon aqueuse.

17. Utilisation de pseudo-réseaux ou micro- et/ou nanoparticules, fabriqués selon l'une des revendications 1 à 15 pour la fabrication de préparations pharmaceutiques.

18. Utilisation selon la revendication 17, caractérisée en ce que dans les pseudo-réseaux, la micro- et/ou nanoparticule, sont incorporés des médicaments.

19. Utilisation selon la revendication 17 ou 18, caractérisée en ce que les préparations pharmaceutiques sont des films par diffusion.
